# EUROPEAN PATENT APPLICATION

(11) **EP 4 756 837 A1**
(43) Date of publication of application: **10.06.2026**
(21) Application number: 24849297.7
(22) Date of filing: 02.08.2024
(51) Int. Cl.: G16H 50/30, A61B 5/107, A61B 6/46

(54) **RISK EVALUATION DEVICE, RISK EVALUATION PROGRAM, AND RISK EVALUATION METHOD**

(30) Priority: 03.08.2023 JP 2023127065
(71) Applicant: Omiya City Clinic, Saitama-shi, Saitama 330-8669 (JP)
(72) Inventor: NAKAGAWA Ryo, Saitama-shi, Saitama 330-8669 (JP)
(74) Representative: Mullholland, Lewis Paul
(86) International application number: PCT/JP2024/027726
(87) International publication number: WO 2025/028652

(57) **Abstract**

Provided is a risk evaluation apparatus capable of easily, quickly, and accurately evaluating a risk of a locomotive syndrome.

A risk evaluation apparatus 10 according to a first embodiment of the present invention includes an acquirer 101 that acquires first information correlated with a fat mass of a body and second information correlated with a muscle mass, and a determiner 102 that determines the risk of the locomotive syndrome based at least on the first information and the second information. According to the risk evaluation apparatus 10, the risk evaluation apparatus capable of easily, quickly, and accurately evaluating the risk of the locomotive syndrome can be provided.

## Description

### Technical Field

The present invention relates to a risk evaluation apparatus, a risk evaluation program, and a risk evaluation method for evaluating the risk of a locomotive syndrome.

### Background Art

With super-aging, a locomotive syndrome issue has been recently taken seriously. A locomotive syndrome (abbreviated as LS) is defined as "a condition of reduced mobility due to impairment of locomotive organs" according to the Japanese Orthopaedic Association. With progress in the LS, the need for care may increase in the future, and for this reason, it is significantly important to evaluate the risk of the LS.

Conventionally, there has been known a technique of evaluating the risk of the locomotive syndrome using a plurality of wearable sensors (see, for example, Patent Literature 1).

### Citation List

### Patent Literature

PTL 1: JP2018-114021A

### Summary of Invention

### Technical Problem

However, in the conventional technique, a subject needs to walk with the plurality of sensors attached to the subject, and there is still room for improvement in easy, quick, and accurate evaluation of the risk of the locomotive syndrome.

For this reason, a main object of the present invention is to provide a risk evaluation apparatus capable of easily, quickly, and accurately evaluating the risk of the locomotive syndrome.

### Solution to Problem

The present invention provides a risk evaluation apparatus including
an acquirer that acquires first information correlated with a fat mass of a body and second information correlated with a muscle mass, and
a determiner that determines the risk of a locomotive syndrome based at least on the first information and the second information.

The fat may be the visceral fat, and the muscle may be the iliopsoas muscle.

The acquirer may acquire the first information and the second information from body image information indicating an image of an internal structure of the body.

The body image information may be a two-dimensional image obtained by imaging a predetermined position of the body, the acquirer may calculate an area X of the visceral fat as the first information from the two-dimensional image, and calculate an area Y of the iliopsoas muscle as the second information from the two-dimensional image, and the determiner may make the determination based on the area X and the area Y.

The determiner may make the determination using an area ratio between the area X and the area Y.

The predetermined position may be the position of the navel of the body.

The two-dimensional image may be a body tomographic image obtained by computed tomography (CT).

The determiner may categorize the risk into a plurality of categories corresponding to a plurality of category indicators by the area ratio and the degree of obesity of the body, and determine the risk.

The plurality of category indicators may include the following four category indicators:
(1) low degree of obesity, low X/Y;
(2) high degree of obesity, low X/Y;
(3) low degree of obesity, high X/Y; and
(4) high degree of obesity, high X/Y.

The risk evaluation apparatus may further include a storage that stores improvement method information on the risk, which is adapted to each of the plurality of categories, and the improvement method information adapted to the category determined by the determiner may be output.

The acquirer may acquire the first information and the second information from a result of measurement obtained on the body with a body composition monitor.

The first information may include a body fat percentage.

The second information may include a leg muscle mass and/or an SMI.

The acquirer may receive the first information and the second information or information including the first information and the second information via a network.

The determiner may transmit a risk determination result via a network.

The determiner may not only determine the risk of the locomotive syndrome, but also determine a life style-related disease risk.

The present invention also provides a risk evaluation program causing a computer to execute
a process of acquiring first information correlated with the visceral fat mass of a body and second information correlated with the iliopsoas muscle mass, and
a process of determining the risk of a locomotive syndrome based at least on the first information and the second information.

The present invention also provides a risk evaluation method including
a step of acquiring first information correlated with the visceral fat mass of a body and second information correlated with the iliopsoas muscle mass, and
a step of determining the risk of a locomotive syndrome based at least on the first information and the second information.

### Brief Description of Drawings

[FIG. 1] FIG. 1 is a table for describing relevance between a result of an LS risk test and parameters of a subject.
[FIG. 2] FIG. 2 is a table for describing relevance between a result of a stand-up test and the parameters of the subject.
[FIG. 3] FIG. 3 is a table for describing relevance between a result of a two-step test and the parameters of the subject.
[FIG. 4] FIG. 4 is a table for describing relevance between a result of a 25-question geriatric locomotive function scale and the parameters of the subject.
[FIG. 5] FIG. 5 is a table showing the degree of the relevance between the result of the LS risk test and the parameters of the subject.
[FIG. 6] FIG. 6 is a graph showing statistical distribution of Visceral Fat/Iliopsoas Muscle.
[FIG. 7] FIG. 7 is a table for describing relevance between the result of the LS risk test, an actual age, and Visceral Fat/Iliopsoas Muscle.
[FIG. 8] FIG. 8 is a table for describing relevance between the result of the stand-up test, the actual age, and Visceral Fat/Iliopsoas Muscle.
[FIG. 9] FIG. 9 is a table for describing relevance between the result of the two-step test, the actual age, and Visceral Fat/Iliopsoas Muscle.
[FIG. 10] FIG. 10 is a table for describing relevance between the result of the 25-question geriatric locomotive function scale, the actual age, and Visceral Fat/Iliopsoas Muscle.
[FIG. 11] FIG. 11 is a graph for describing LS category indicators and category examples.
[FIG. 12] FIG. 12 is a graph showing the percentage of persons with impaired glucose tolerance by age when these persons are categorized into groups according to the degree of obesity and an LS stage (qualitative).
[FIG. 13] FIG. 13 is a graph showing the percentage of persons with impaired glucose tolerance by age when these persons are categorized into groups according to the degree of obesity and Visceral Fat/Iliopsoas Muscle (quantitative).
[FIG. 14] FIG. 14 is a block diagram showing the configuration of a risk evaluation apparatus according to a first embodiment of the present invention.
[FIG. 15] FIG. 15 is a view showing one example of body image information.
[FIG. 16] FIG. 16 is a skeletal muscle view showing an example of a section of a body including the psoas muscle thereof.
[FIG. 17] FIG. 17 is a flowchart for describing operation of the risk evaluation apparatus according to the first embodiment of the present invention.
[FIG. 18] FIG. 18 is a block diagram showing the configuration of a risk evaluation apparatus according to a second embodiment of the present invention.
[FIG. 19] FIG. 19 is a block diagram showing the configuration of a risk evaluation apparatus according to a third embodiment of the present invention.
[FIG. 20] FIG. 20 is a flowchart for describing operation of the risk evaluation apparatus according to the third embodiment of the present invention.
[FIG. 21] FIG. 21 is a block diagram showing the configuration of a risk evaluation apparatus according to a fourth embodiment of the present invention.
[FIG. 22] FIG. 22 is a block diagram showing the configuration of a risk evaluation apparatus according to a fifth embodiment of the present invention.
[FIG. 23] FIG. 23 is a graph for describing other example of the LS category indicators and category examples.
[FIG. 24] FIG. 24 is a table showing relevance between the 25-question geriatric locomotive function scale and a measurement result of a body composition monitor.
[FIG. 25] FIG. 25 is a table showing relevance between the stand-up test and the measurement result of the body composition monitor.
[FIG. 26] FIG. 26 is a table showing relevance between the two-step test and the measurement result of the body composition monitor.

### Description of Embodiments

Hereinafter, preferred embodiments of the present invention will be described in detail with reference to the attached drawings. Note that components having substantially the same functional configurations are denoted by the same reference numerals in the present specification and the drawings and overlapping description thereof will be omitted. The embodiments described below are representative embodiments of the present invention, and are not intended to interpret the scope of the present invention as a narrower scope. In the present specification, even in a case where it is described that each of a risk evaluation apparatus, a risk evaluation program, and a risk evaluation method according to the present invention has a plurality of effects, each of the risk evaluation apparatus, the risk evaluation program, and the risk evaluation method according to the present invention is only required to have at least one effect. The effects described in the present specification are merely examples and are not limited, and other effects may be produced.

The description will be made in the following order:
0. Outline of Present Invention;
1. Risk Evaluation Apparatus according to First Embodiment of Present invention;
2. Risk Evaluation Apparatus according to Second Embodiment of Present invention;
3. Risk Evaluation Apparatus according to Third Embodiment of Present invention;
4. Risk Evaluation Apparatus according to Fourth Embodiment of Present invention;
5. Risk Evaluation Apparatus according to Fifth Embodiment of Present invention; and
6. Modifications of Present Invention

### <0. Outline of Present Invention>

A locomotive syndrome (LS) is a condition of reduced mobility of a body due to impairment of locomotive organs such as bones, joints, muscles, and nerves, and is one cause of need for care. It has been pointed out that the LS is related to life style-related diseases such as a high-blood pressure and diabetes. For this reason, it is significantly important for extension of a healthy lifespan (period in which a person can live a daily life in good health) to prevent the LS, prevent progression of the LS, and preferably improve the LS. As a technique of checking the risk of the LS (risk related to the LS from a body condition not affected with the LS to a body condition which is affected with the LS, but is in a stage before a stage in need of care, hereinafter also referred to as an "LS risk"), an LS risk test recommended by the Japanese Orthopaedic Association has been known. The LS risk test includes a stand-up test, a two-step test, and a 25-question geriatric locomotive function scale. Of these tests, the stand-up test and the two-step test are tests conducted while a subject is moving one's body, and are higher in objectivity (reliability) than the 25-question geriatric locomotive function scale in which a subject answers 25 questions about malfunction of the locomotive organs. However, in the tests involving the body movement, the subject needs to actually move one's body in the presence of an examiner (grader) at a testing facility, and for this reason, these tests still have room for improvement in easiness/promptness and in accuracy because these tests may be dependent on a physical condition or the like at the time of conducting the test on the subject.

For these reasons, the inventor(s) has conducted intensive study on relevance between the locomotive syndrome (LS) and parameters of the subject, and found out that the LS and certain parameters (specifically, fat mass (for example, visceral fat mass) and muscle mass (for example, iliopsoas muscle mass)) of the subjects are closely relevant to each other. This is a new finding obtained by the inventor (s) .

Then, the inventor(s) has developed the risk evaluation apparatus according to the present invention as a technical idea obtained by embodying this new finding. According to the risk evaluation apparatus of the present invention, the locomotive syndrome can be easily, quickly, and accurately evaluated.

Further, the inventor(s) has also developed the risk evaluation program and the risk evaluation method according the present invention as technical ideas obtained by embodying the new finding above.

The present invention can easily, quickly, and accurately evaluate the LS risk, and is so groundbreaking that the present invention can coexist with the LS risk test in the future or can be replaced with the LS risk test. The inventor(s) has great expectation that a wide range of use of the present invention at healthcare facilities and the like will be an opportunity for recognizing the LS risk at an earlier stage, driving improvement of the LS risk, and therefore extending the healthy lifespan in a super-aging society.

The inventor(s) has completed the present invention in such a manner that the inventor(s) thoroughly analyzes which one of the parameters of the subject is related to an LS stage which is a result of the LS risk test and how much such a parameter is related to the LS stage and provides feedback on the analysis result. Thus, the analysis result about the relevance between the result of the LS risk test and the parameters of the subject will be first described.

### (Relevance between Result of LS Risk Test and Parameters of Subject)

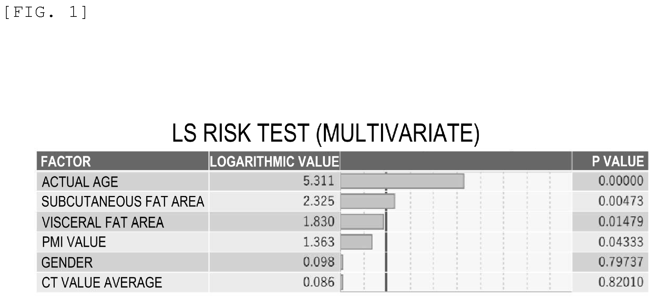

FIG. 1 is a table showing the relevance between the result (LS stage) of the LS risk test (general) and the parameters of the subject. According to the LS risk test, the LS risk can be determined as a non-LS state, LS stage 1 in which the LS has started, LS stage 2 in which the LS has progressed, and LS stage 3 in which the LS has further progressed and interferes with social participation. As shown in FIG. 1, data obtained by aggregating correlation between the result of the LS risk test conducted on each of many subjects in the past and the parameters of such a subject shows that the result of the LS risk test (general) is relevant to each parameter (LS factor) of the subject including an actual age, a subcutaneous fat area, a visceral fat area, a PMI value, a gender, and a CT value average. In FIG. 1, as a logarithmic value for the parameter increases and a P value for the parameter decreases, the parameter is more relevant to the result of the LS risk test. FIG. 1 shows that particularly the actual age, subcutaneous fat area, visceral fat area, PMI value of the subject are significantly relevant to the result of the LS risk test (general). Here, the PMI value above is a value obtained in such a manner that the area of the iliopsoas muscle in a body tomographic image (for example, CT image) of the position (in the vicinity of the third lumbar vertebra) of the navel of the subject is divided by the square of a body height (the same also applies hereinafter). The subcutaneous fat area above is the area of the subcutaneous fat in the body tomographic image (the same also applies hereinafter). The visceral fat area above is the area of the visceral fat in the body tomographic image (the same also applies hereinafter). The CT value average above is the average of the visceral fat areas of all the subjects (the same also applies hereinafter).

### (Relevance between Result of Stand-Up Test and Parameters of Subject)

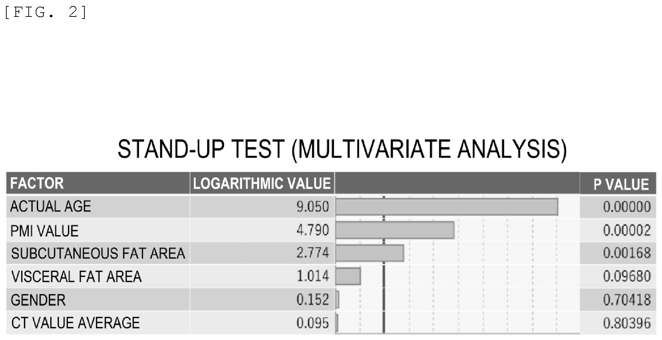

FIG. 2 is a table for describing relevance between the result of the stand-up test and the parameters of the subject. The stand-up test is a test for measuring from what height of a chair the subject can stand up on one leg or both legs. In FIG. 2, as the logarithmic value for the parameter increases and the P value for the parameter decreases, the parameter is more relevant to the result of the stand-up test. FIG. 2 shows that particularly the actual age, PMI value, subcutaneous fat area, and visceral fat area of the subject are greatly relevant to the result of the stand-up test.

### (Relevance between Result of Two-Step Test and Parameters of Subject)

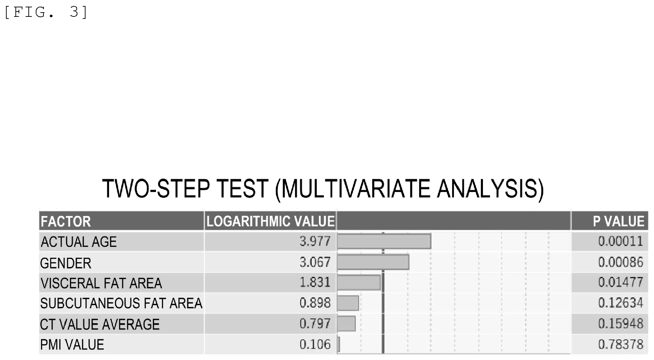

FIG. 3 is a table for describing relevance between the result of the two-step test and the parameters of the subject. The two-step test is a test for measuring a distance by which the subject can walk two steps as large as possible. In FIG. 3, as the logarithmic value for the parameter increases and the P value for the parameter decreases, the parameter is more relevant to the result of the two-step test. FIG. 3 shows that particularly the actual age, gender, and visceral fat area of the subject are greatly relevant to the result of the two-step test.

### (Relevance between Result of 25-Question Geriatric Locomotive Function Scale and Parameters of Subject)

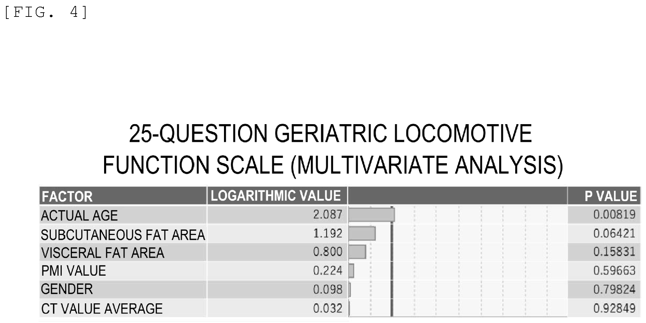

FIG. 4 is a table for describing relevance between the result of the 25-question geriatric locomotive function scale and the parameters of the subject. As described above, the 25-question geriatric locomotive function scale is the test in which the subject answers the 25 questions about malfunction of the locomotive organs. In FIG. 4, as the logarithmic value for the parameter increases and the P value for the parameter decreases, the parameter is more relevant to the result of the 25-question geriatric locomotive function scale. FIG. 4 shows that particularly the actual age, subcutaneous fat area, and visceral fat area of the subject are greatly relevant to the result of the 25-question geriatric locomotive function scale.

### (Overview: Degree of Relevance between Result of LS Risk Test and Parameters of Subject)

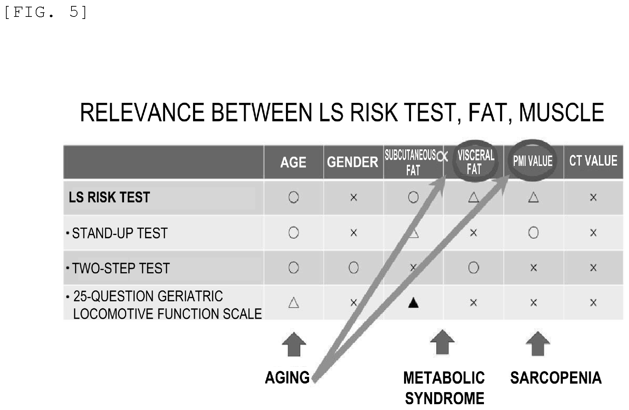

FIG. 5 is a table showing the degree of the relevance between the result of the LS risk test and the parameters of the subject. In FIG. 5, a white circle indicates a high relevance, a white triangle indicates an upper intermediate relevance, a cross mark indicates a low relevance, and a black triangle indicates a lower intermediate relevance. FIG. 5 shows that in addition to the age of the subject, particularly the visceral fat mass and the PMI value are greatly relevant to the result (LS stage) of the LS risk test. Thus, the locomotive syndrome can also be defined as reduced mobility due to combined factors of aging, a metabolic syndrome (dependent on the visceral fat mass), and sarcopenia (dependent on (dependent on the PMI value dependent on the iliopsoas muscle mass).

### (Relationship of Visceral Fat and Iliopsoas Muscle with LS)

As the visceral fat increases in mass, a body weight increases, the subject cannot carry one's body farther, and one step of the subject becomes smaller. This leads to a decrease in the score of the two-step test (increase in the LS stage). As the iliopsoas muscle becomes thinner, the strength to hold one's posture becomes weaker. This leads to a decrease in the score of the stand-up test (increase in the LS stage). For this reason, it can be said that the visceral fat mass is a factor associated with an increase in the LS risk and the iliopsoas muscle mass is a factor associated with a decrease in the LS risk. Thus, the LS risk can be quantitively evaluated using a ratio between the visceral fat mass and the iliopsoas muscle mass. For example, the LS risk increases as Visceral Fat Mass/Iliopsoas Muscle Mass increases, and decreases as Visceral Fat Mass/Iliopsoas Muscle Mass decreases. For example, the LS risk decreases as Iliopsoas Muscle Mass/Visceral Fat Mass increases, and increases as Iliopsoas Muscle Mass/Visceral Fat Mass decreases.

### (Visceral Fat/Iliopsoas Muscle)

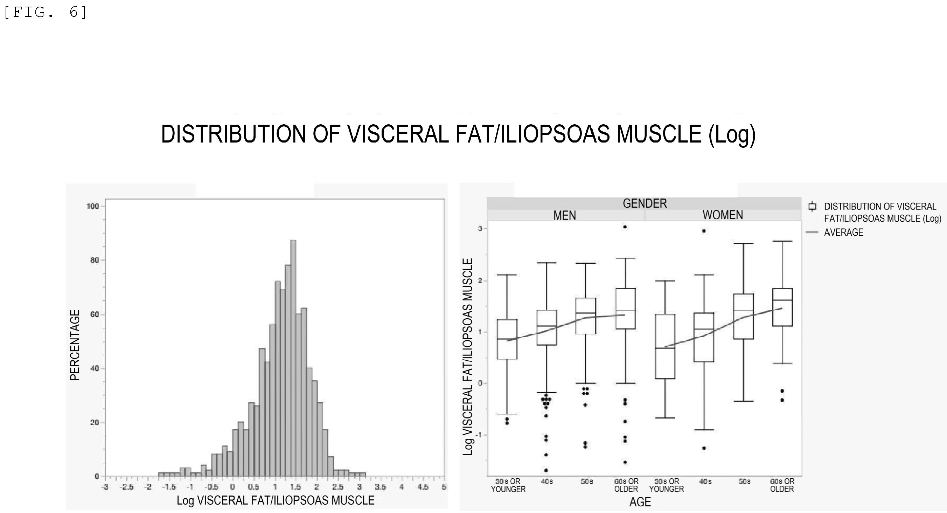

FIG. 6 is a graph showing statistical distribution of Visceral Fat/Iliopsoas Muscle (specifically Visceral Fat Mass/Iliopsoas Muscle Mass, the same also applies hereinafter). The graphs on the left and right sides in FIG. 6 show that Visceral Fat/Iliopsoas Muscle (logarithmic value) shows normal distribution, has almost no difference between men and women, and increases due to aging. Note that Visceral Fat/Iliopsoas Muscle also fluctuates due to a lifestyle habit such as a dietary habit or an exercise routine in addition to aging.

### (Relevance between Result of LS Risk Test and Visceral Fat/Iliopsoas Muscle)

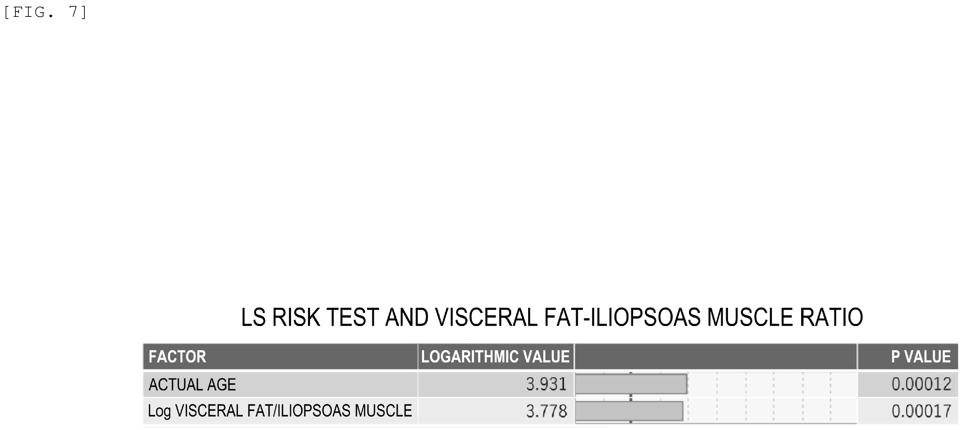

FIG. 7 is a table for describing relevance between the result of the LS risk test (general), the actual age, and Visceral Fat/Iliopsoas Muscle. In FIG. 7, as the logarithmic value for the parameter increases and the P value for the parameter decreases, the parameter is more relevant to the result of the LS risk test. FIG. 7 shows that the relevance of Visceral Fat/Iliopsoas Muscle to the result of the LS risk test (general) is similar to that of the actual age.

### (Relevance between Result of Stand-Up Test and Visceral Fat/Iliopsoas Muscle)

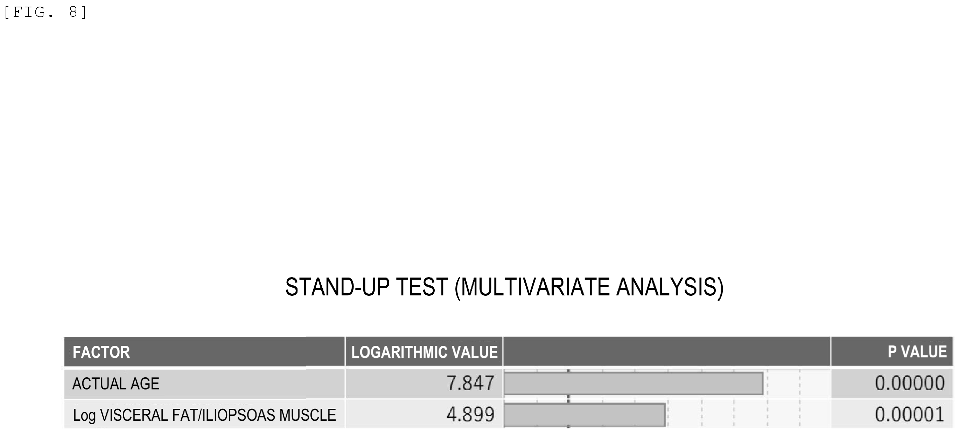

FIG. 8 is a table for describing relevance between the result of the stand-up test, the actual age, and Visceral Fat/Iliopsoas Muscle. In FIG. 8, as the logarithmic value for the parameter increases and the P value for the parameter decreases, the parameter is more relevant to the result of the stand-up test. FIG. 8 shows that the relevance of Visceral Fat/Iliopsoas Muscle to the result of the stand-up test is not as high as that of the actual age, but is significantly high.

### (Relevance between Result of Two-Step Test and Visceral Fat/Iliopsoas Muscle)

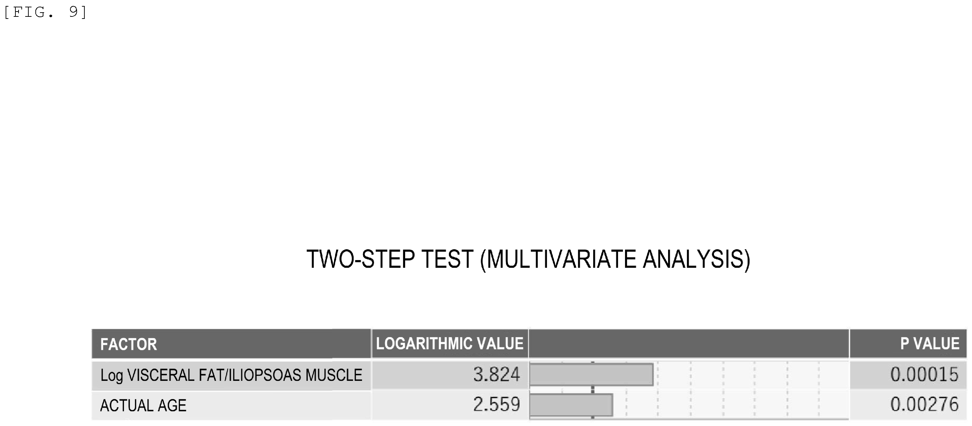

FIG. 9 is a table for describing relevance between the result of the two-step test, the actual age, and Visceral Fat/Iliopsoas Muscle. In FIG. 9, as the logarithmic value for the parameter increases and the P value for the parameter decreases, the parameter is more relevant to the result of the two-step test. FIG. 9 shows that the relevance of Visceral Fat/Iliopsoas Muscle to the result of the two-step test is significantly higher than that of the actual age.

### (Relevance between Result of 25-Question Geriatric Locomotive Function Scale and Visceral Fat/Iliopsoas Muscle)

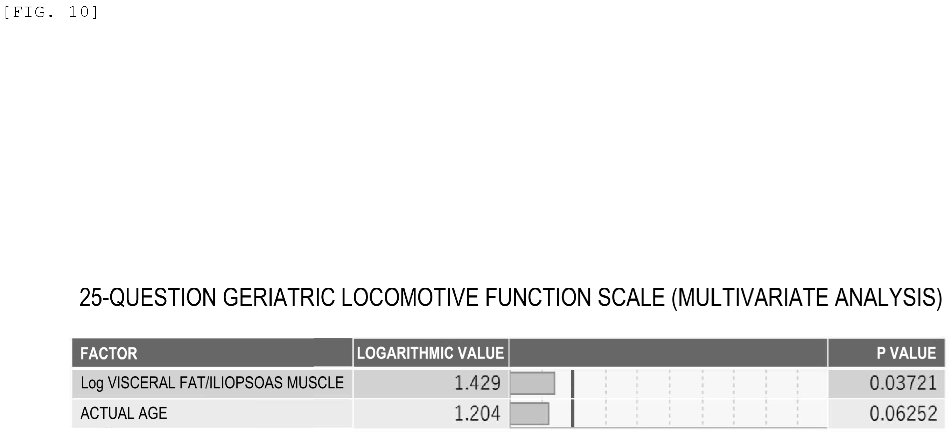

FIG. 10 is a table for describing relevance between the result of the 25-question geriatric locomotive function scale, the actual age, and Visceral Fat/Iliopsoas Muscle. In FIG. 10, as the logarithmic value for the parameter increases and the P value for the parameter decreases, the parameter is more relevant to the result of the 25-question geriatric locomotive function scale. FIG. 10 shows that the relevance of Visceral Fat/Iliopsoas Muscle to the result of the 25-question geriatric locomotive function scale exceeds that of the actual age.

As described above, it shows that the relevance of Visceral Fat/Iliopsoas Muscle to the result of the LS risk test is equivalent to that of the actual age.

### (LS Categories)

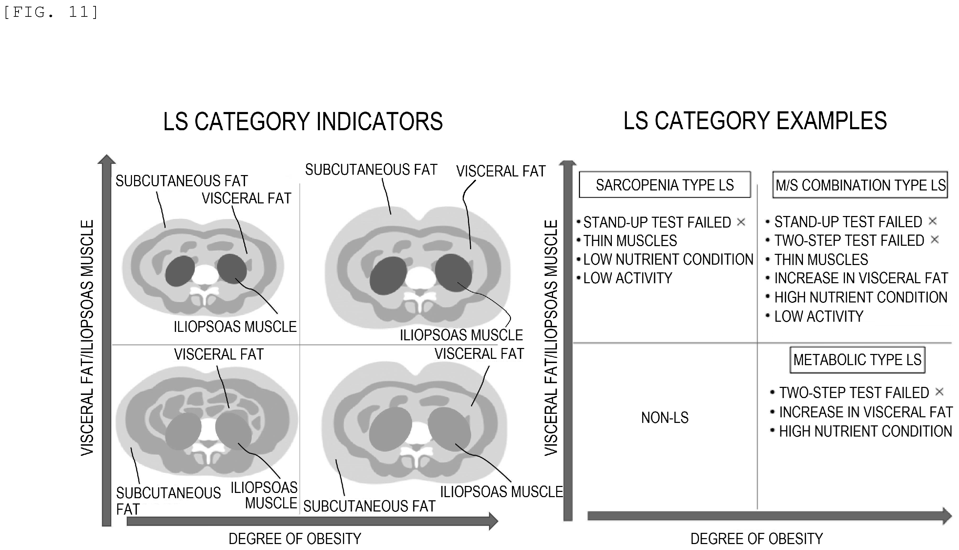

FIG. 11 is a graph showing LS category indicators and category examples. As shown in the graph on the left side in FIG. 11, for example, the following category indicators for categorizing the LS risk can be obtained for LS categories from the degree of obesity and Visceral Fat Area/Iliopsoas Muscle Area (X/Y). The degree of obesity is defined, for example, by a body mass index (BMI) value.
(1) low degree of obesity, low X/Y;
(2) high degree of obesity, low X/Y;
(3) low degree of obesity, high X/Y; and
(4) high degree of obesity, high X/Y.

Four quadrants of the graph on the right side in FIG. 11 correspond to four quadrants of the graph on the left side in FIG. 11 in one-to-one correspondence. As one example, the LS risk is categorized as a non-LS (not applicable to the LS) according to the category indicator (1) above, is categorized as a metabolic type LS according to the category indicator (2) above, is categorized as a sarcopenia type LS according to the category indicator (3) above, and is categorized as a metabolic/sarcopenia (M/S) combination type LS according to the category indicator (4) above. The metabolic type LS is characterized by failure of the two-step test, an increase in the visceral fat, a high nutrient condition, and the like. The sarcopenia type LS is characterized by failure of the stand-up test, thin muscles, a low nutrient condition, low activity, and the like. The M/S combination type LS is characterized by failure of the stand-up test, failure of the two-step test, thin muscles, an increase in the visceral fat, a high nutrient condition, low activity, and the like.

### (Relationship of Degree of Obesity and LS Stage with Impaired Glucose Tolerance)

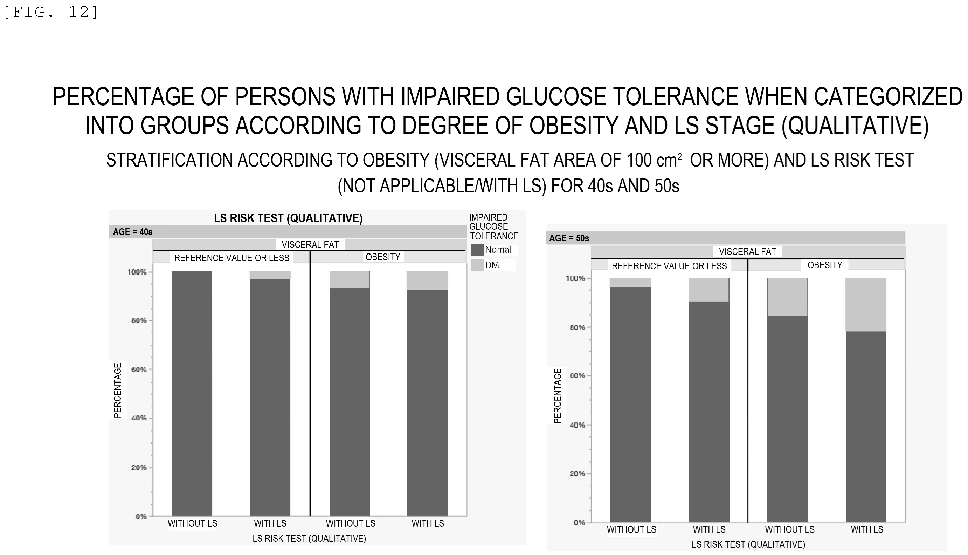

FIG. 12 is a graph showing an example of the percentage of persons with impaired glucose tolerance by age when these persons are categorized into groups according to the degree of obesity and the LS stage (qualitative). The degree of obesity is expressed by the body mass index (BMI).

According to the graph on the left side in FIG. 12, for the persons in one's 40s, the percentage (DM) of the persons with impaired glucose tolerance is 0% in the case of persons without the LS and about 5% in the case of persons with the LS in a group with a visceral fat of a reference value or less. According to the graph on the left side in FIG. 12, for the persons in one's 40s, the percentage (DM) of the persons with impaired glucose tolerance is about 10% regardless of with or without the LS in an obesity group with a visceral fat of exceeding the reference value.

According to the graph on the right side in FIG. 12, for the persons in one's 50s, the percentage (DM) of the persons with impaired glucose tolerance is about 5% in the case of persons without the LS and about 10% in the case of persons with the LS in a group with a visceral fat of a reference value or less. According to the graph on the right side in FIG. 12, for the persons in one's 50s, the percentage (DM) of the persons with impaired glucose tolerance is about 15% in the case of persons without the LS and about 20% in the case of persons with the LS in an obesity group with a visceral fat of exceeding the reference value.

### (Relationship of Degree of Obesity and Visceral Fat/Iliopsoas Muscle with Impaired Glucose Tolerance)

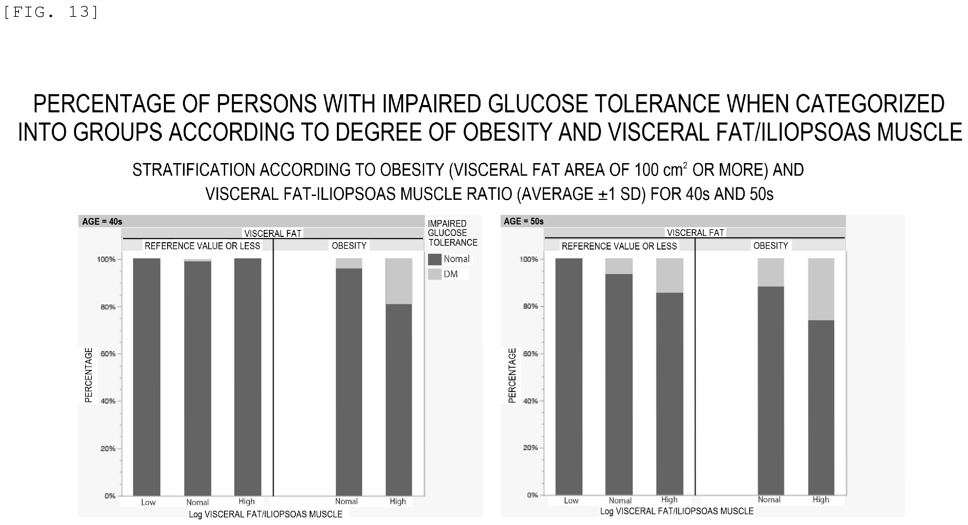

FIG. 13 is a graph showing an example of the percentage of persons with impaired glucose tolerance by age when these persons are categorized into groups according to the degree of obesity and Visceral Fat/Iliopsoas Muscle (quantitative).

According to the graph on the left side in FIG. 13, for the persons in one's 40s, the percentage (DM) of the persons with impaired glucose tolerance is 0% both in cases where Visceral Fat/Iliopsoas Muscle is low (Low) and high (High), but on the other hand, is about 2% in a case where Visceral Fat/Iliopsoas Muscle is intermediate (Normal) in a group with a visceral fat of a reference value or less. According to the graph on the left side in FIG. 13, for the persons in one's 40s, the percentage (DM) of the persons with impaired glucose tolerance is about 5% in a case where Visceral Fat/Iliopsoas Muscle is intermediate (Normal) and about 20% in a case where Visceral Fat/Iliopsoas Muscle is high (High) in an obesity group with a visceral fat of exceeding the reference value.

According to the graph on the right side in FIG. 13, for the persons in one's 50s, the percentage (DM) of the persons with impaired glucose tolerance is 0% in a case where Visceral Fat/Iliopsoas Muscle is low (Low), about 5% in a case where Visceral Fat/Iliopsoas Muscle is intermediate (Normal), and about 15% in a case where Visceral Fat/Iliopsoas Muscle is high (High) in a group with a visceral fat of a reference value or less. According to the graph on the right side in FIG. 13, for the persons in one's 50s, the percentage (DM) of the persons with impaired glucose tolerance is about 10% in a case where Visceral Fat/Iliopsoas Muscle is intermediate (Normal) and about 30% in a case where Visceral Fat/Iliopsoas Muscle is high (High) in an obesity group with a visceral fat of exceeding the reference value.

As a result of comparison between the example of FIG. 12 and the example of FIG. 13, it shows that the combination of the degree of obesity and Visceral Fat/Iliopsoas Muscle shows identifiability to determine the presence or absence of the impaired glucose tolerance, which is equal to or higher than that of the combination of the degree of obesity and the LS stage (result of the LS risk test).

Hereinafter, the risk evaluation apparatus according to the present invention will be described in detail with reference to some embodiments.

### <1. Risk Evaluation Apparatus according to First Embodiment of Present Invention>

### (Configuration of Risk Evaluation Apparatus)

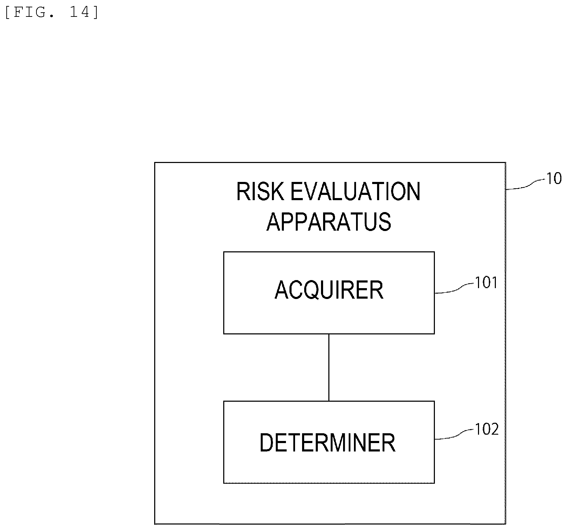

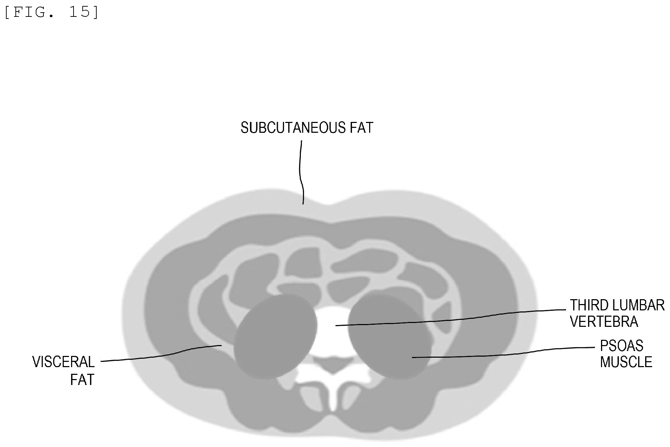

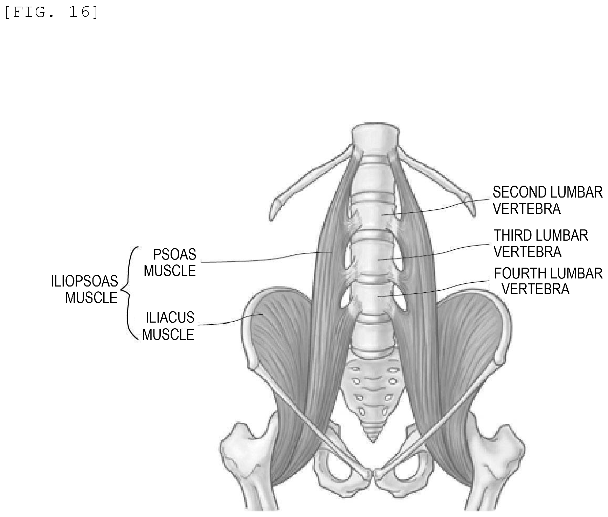

Hereinafter, the configuration of a risk evaluation apparatus 10 according to a first embodiment of the present invention will be described. FIG. 14 is a block diagram showing the configuration of the risk evaluation apparatus 10 according to the first embodiment of the present invention. FIG. 15 is a view showing one example of body image information. FIG. 16 is a skeletal muscle view showing an example of a section of a body including the psoas muscle thereof.

The risk evaluation apparatus 10 is placed at a healthcare facility or the like, as one example.

As shown in FIG. 14, the risk evaluation apparatus 10 includes an acquirer 101 that acquires first information correlated with the visceral fat mass of the body and second information correlated with the iliopsoas muscle mass, and a determiner 102 that determines the risk (LS risk) of the locomotive syndrome based at least on the first information and the second information. The acquirer 101 is implemented, for example, by hardware such as a central processing unit (CPU), a graphics processing unit (GPU), or a chipset. The determiner 102 is implemented, for example, by hardware such as a comparator or a chipset.

As one example, the acquirer 101 acquires the first information and the second information above from the body image information indicating an image of the internal structure of the body.

The body image information is, as one example, a two-dimensional image (specifically two-dimensional image data) (see FIG. 15) obtained by imaging a predetermined position of the body. The predetermined position is, for example, the position of the navel of the body. Specifically, the predetermined position is, for example, a position within a region from the upper end of the second lumbar vertebra to the lower end of the fourth lumbar vertebra as shown in FIG. 16, and is preferably a position within a region from the upper end to the lower end of the third lumbar vertebra. As one example, the two-dimensional image shown in FIG. 15 shows the psoas muscle of the iliopsoas muscle and the third lumbar vertebra.

As one example, the two-dimensional image is a body tomographic image (for example, CT image) obtained by computed tomography (CT). Note that the two-dimensional image may be a body tomographic image (for example, MRI image) obtained by magnetic resonance imaging (MRI).

As one example, the acquirer 101 calculates a visceral fat area X as the first information above from the two-dimensional image, and calculates an iliopsoas muscle area Y as the second information above from the two-dimensional image. The acquirer 101 further calculates the ratio between the areas X, Y. As the area ratio, X/Y may be calculated, or Y/X may be calculated. As one example, the acquirer 101 includes an image processor and an arithmetic processor. In the acquirer 101, the image processor identifies each of the visceral fat region and the iliopsoas muscle region in the two-dimensional image, for example, by image processing such as edge extraction or contour extraction, and the arithmetic processor calculates the visceral fat area X and the iliopsoas muscle area Y.

The determiner 102 determines the LS risk based on the area X and the area Y. Specifically, the determiner 102 determines the LS risk using the ratio between the area X and the area Y, and outputs the determination result.

Specifically, as one example, the determiner 102 causes the comparator to compare X/Y with a predetermined threshold, and determines that no LS is present in a case where X/Y is the predetermined threshold or less (the output of the comparator is Low) and determines that the LS is present in a case where X/Y exceeds the threshold (the output of the comparator is High). That is, the determination made here is simple determination on the presence or absence of the LS.

### (Operation of Risk Evaluation Apparatus)

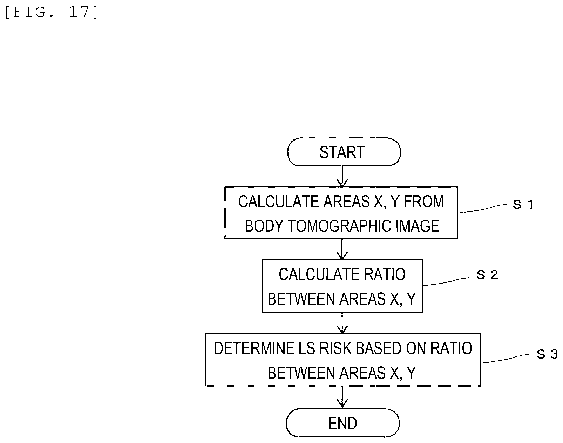

Hereinafter, operation of the risk evaluation apparatus 10 according to the first embodiment of the present invention will be described. FIG. 17 is a flowchart for describing the operation of the risk evaluation apparatus 10 according to the first embodiment of the present invention. Using the risk evaluation apparatus 10, one example of the risk evaluation method according to the present invention can be implemented. The flowchart of FIG. 17 corresponds to a risk evaluation program which is a program to be executed by the CPU (computer) of the risk evaluation apparatus 10.

First, in Step S1, the acquirer 101 calculates the visceral fat area X and the iliopsoas muscle area Y from the body tomographic image. The body tomographic image is, for example, a CT image of the position of the navel of a subject provided directly from the subject or via the healthcare facility. Specifically, the acquirer 101 causes the image processor to extract the visceral fat and the iliopsoas muscle from the CT image, and causes the arithmetic processor to calculate the visceral fat area X and the iliopsoas muscle area Y.

Subsequently, in Step S2, the acquirer 101 calculates the ratio between the areas X, Y. Specifically, the acquirer 101 causes the arithmetic processor to calculate, for example, X/Y.

Finally, in Step S3, the determiner 102 determines the LS risk based on the ratio between the areas X, Y (for example, X/Y), and outputs the determination result. Specifically, the determiner 102 causes the comparator to compare X/Y with the predetermined threshold, and outputs the determination result indicating the absence of the LS in a case where X/Y is the threshold or less and outputs the determination result indicating the presence of the LS in a case where X/Y exceeds the threshold. A method for outputting the LS risk determination result from the determiner 102 includes output via a monitor, audio output, printing out, and the like.

### (Effects of Risk Evaluation Apparatus, Risk Evaluation Program, and Risk Evaluation Method)

The risk evaluation apparatus 10 according to the first embodiment as described above includes the acquirer 101 that acquires the first information correlated with the visceral fat mass of the body and the second information correlated with the iliopsoas muscle mass, and the determiner 102 that determines the risk (LS risk) of the locomotive syndrome based at least on the first information and the second information.

According to the risk evaluation apparatus 10, the LS risk can be determined based on the first information and the second information, and therefore, the LS risk of the subject can be evaluated with favorable accuracy without the subject actually having exercise at a testing facility, as compared with the LS risk test and the related art (for example, Patent Literature 1) using a plurality of wearable sensors.

As a result, according to the risk evaluation apparatus 10, a risk evaluation apparatus capable of easily, quickly, and accurately evaluating the LS risk can be provided.

For example, evaluation of the LS risk with the risk evaluation apparatus 10 is introduced as an examination item of a comprehensive medical examination, which is significantly meaningful in that a subject of the comprehensive medical examination can also receive the LS risk evaluation result. At this time, for example, a CT image obtained by imaging at CT scan in the comprehensive medical examination is also used for the LS risk evaluation, which is efficient and can provide the subject with the LS risk evaluation result (evaluation result meaningful for the subject) from the latest CT image. Further, the LS risk evaluation result can be easily and quickly obtained, for example, in the comprehensive medical examination, which is also significantly meaningful in that the waiting time and examination time for obtaining the LS risk evaluation are not required.

The risk evaluation apparatus 10 according to the first embodiment of the present invention is operated by the risk evaluation program causing the computer to execute a process of acquiring the first information correlated with the visceral fat mass of the body and the second information correlated with the iliopsoas muscle mass and a process of determining the risk of the locomotive syndrome based at least on the first information and the second information.

According to the risk evaluation program, the risk evaluation apparatus 10 can easily, quickly, and accurately evaluate the LS risk.

Using the risk evaluation apparatus 10 according to the first embodiment of the present invention, the risk evaluation method is implemented, which includes a step of acquiring the first information correlated with the visceral fat mass of the body and the second information correlated with the iliopsoas muscle mass and a step of determining the risk of the locomotive syndrome based at least on the first information and the second information.

According to the risk evaluation method, the LS risk can be easily, quickly, and accurately evaluated.

### <2. Risk Evaluation Apparatus according to Second Embodiment of Present Invention>

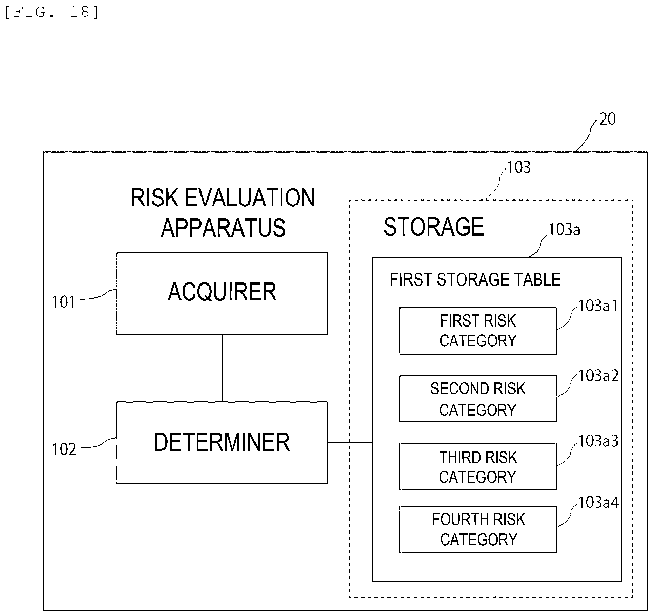

Hereinafter, a risk evaluation apparatus according to a second embodiment of the present invention will be described with reference to FIG. 18. FIG. 18 is a block diagram showing the configuration of a risk evaluation apparatus 20 according to the second embodiment of the present invention.

The risk evaluation apparatus 20 has a configuration similar to that of the risk evaluation apparatus 10 according to the first embodiment, except that the risk evaluation apparatus 20 further includes a storage 103.

The storage 103 includes at least one storage medium such as a read only memory (ROM), a random access memory (RAM), a flash memory, or a hard drive.

As one example, in the risk evaluation apparatus 20, the determiner 102 categorizes the LS risk into a plurality of risk categories corresponding to a plurality of category indicators by the ratio between the areas X, Y and the degree of obesity (for example, BMI value) of the body, and determines the LS risk (see FIG. 11). The plurality of category indicators include, for example, the following four category indicators (1) to (4):
(1) low degree of obesity, low X/Y;
(2) high degree of obesity, low X/Y;
(3) low degree of obesity, high X/Y; and
(4) high degree of obesity, high X/Y.

Here, the determiner 102 has a first comparator that compares the degree of obesity with a first threshold Th1, and a second comparator that compares X/Y with a second threshold Th2. The first comparator outputs Low (low degree of obesity) in a case where the degree of obesity is the first threshold Th1 or less, and outputs High (high degree of obesity) in a case where the degree of obesity exceeds the first threshold Th1. The second comparator outputs Low (low X/Y) in a case where X/Y is the second threshold Th2 or less, and outputs High (high X/Y) in a case where X/Y exceeds the second threshold Th2. The determiner 102 reads the risk category corresponding to the category indicator determined based on the outputs of the first comparator and the second comparator from a first storage table 103a of the storage 103.

The degree of obesity (for example, BMI value) is provided directly from the subject or via the healthcare facility, and is preferably the latest degree.

The first storage table 103a of the storage 103 saves (stores) the plurality (for example, four) of risk categories (for example, first to fourth risk categories 103a1 to 103a4) corresponding to the plurality (for example, four) of category indicators. As one example, the first to fourth risk categories 103a1 to 103a4 are four risk categories (non-LS, metabolic type LS, sarcopenia type LS, and metabolic/sarcopenia (M/S) combination type LS, see the graph on the right side in FIG. 11) individually corresponding to the four category indicators (1) to (4) above.

As one example, in the risk evaluation apparatus 20, the determiner 102 categorizes the LS risk of the subject into the first to fourth risk categories 103a1 to 103a4 based on the four category indicators (1) to (4) above, and determines the LS risk.

For example, in a case where the degree of obesity is the first threshold Th1 or less and X/Y is the second threshold Th2 or less (applicable to the category indicator (1)), the determiner 102 reads the first risk category 103a1 (non-LS) corresponding to the category indicator (1) from the first storage table 103a, and outputs such a category as the LS risk determination result.

For example, in a case where the degree of obesity exceeds the first threshold Th1 and X/Y is the second threshold Th2 or less (applicable to the category indicator (2)), the determiner 102 reads the second risk category 103a2 (metabolic type LS) corresponding to the category indicator (2) from the first storage table 103a, and outputs such a category as the LS risk determination result.

For example, in a case where the degree of obesity is the first threshold Th1 or less and X/Y exceeds the second threshold Th2 (applicable to the category indicator (3)), the determiner 102 reads the third risk category 103a3 (sarcopenia type LS) corresponding to the category indicator (3) from the first storage table 103a, and outputs such a category as the LS risk determination result.

For example, in a case where the degree of obesity exceeds the first threshold Th1 and X/Y exceeds the second threshold Th2 (applicable to the category indicator (4)), the determiner 102 reads the fourth risk category 103a4 (M/S combination type LS) corresponding to the category indicator (4) from the first storage table 103a, and outputs such a category as the LS risk determination result.

According to the risk evaluation apparatus 20 described above, effects similar to those of the risk evaluation apparatus 10 according to the first embodiment can be obtained, and more detailed LS risk information can be provided to the subject.

Further, a risk evaluation program for operating the risk evaluation apparatus 20 and a risk evaluation method implemented using the risk evaluation apparatus 20 can also be provided.

### <3. Risk Evaluation Apparatus according to Third Embodiment of Present Invention>

### (Configuration of Risk Evaluation Apparatus)

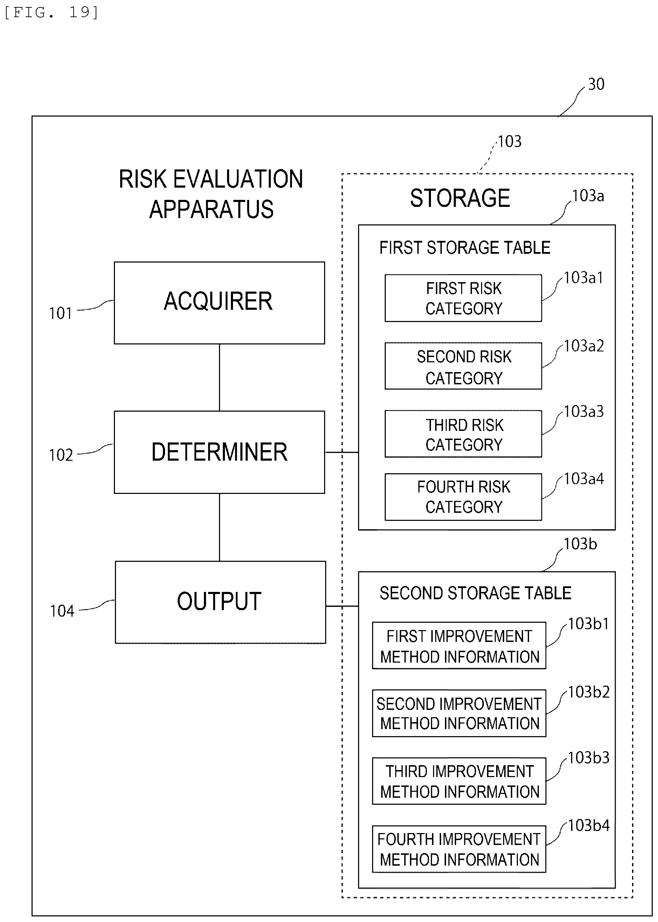

Hereinafter, a risk evaluation apparatus 30 according to a third embodiment of the present invention will be described with reference to FIG. 19. FIG. 19 is a block diagram showing the configuration of the risk evaluation apparatus 30 according to the third embodiment of the present invention.

The risk evaluation apparatus 30 has a configuration similar to that of the risk evaluation apparatus 20 according to the second embodiment, except that the risk evaluation apparatus 30 further includes an output 104 and the storage 103 has a second storage table 103b in addition to the first storage table 103a.

Each of the first storage table 103a and the second storage table 103b may be saved in different storage areas of the same storage medium, or be saved in different storage media.

The second storage table 103b saves (stores) improvement method information on the LS risk, which is adapted to each of a plurality (for example, four) of risk categories. Specifically, the second storage table 103b saves first improvement method information 103b1 adapted to the first risk category 103a1, second improvement method information 103b2 adapted to the second risk category 103a2, third improvement method information 103b3 adapted to the third risk category 103a3, and fourth improvement method information 103b4 adapted to the fourth risk category 103a4.

Specific examples of the first improvement method information 103b1 include a recommendation indicating that the subject is not currently affected with the LS, but needs to review a dietary habit and an exercise routine in order to avoid the risk of being affected with the LS in the future.

Specific examples of the second improvement method information 103b2 include an improvement idea for a dietary habit (for example, setting the upper limit of calories to be taken per day) and an improvement idea for an exercise routine (for example, having aerobic exercise for several tens of minutes or for several hours every day or every other day) because the subject currently falls into the metabolic type LS.

Specific examples of the third improvement method information 103b3 include a specific proposal for improving a dietary habit (for example, setting the upper limit of calories to be taken) and a specific proposal for improving a current exercise routine (for example, having anaerobic exercise for several tens of minutes or for several hours every day or every other day) because the subject currently falls into the sarcopenia type LS.

Specific examples of the fourth improvement method information 103b4 include a specific proposal for improving a dietary habit (for example, setting the upper and lower limits of calories to be taken) and a specific proposal for improving a current exercise routine (for example, having aerobic exercise and anaerobic exercise for several tens of minutes or for several hours every day or every other day) because the subject currently falls into the M/S combination type LS.

The determiner 102 transmits the LS risk determination result (for example, any of the first to fourth risk categories 103a1 to 103a4) to the output 104.

The output 104 outputs the risk category determined by the determiner 102 and the improvement method information adapted to such a risk category. A method for outputting the risk category and the improvement method information from the output 104 includes output via a monitor, audio output, printing out, and the like. Note that the determiner 102 may also fulfill at least some of the functions of the output 104.

### (Operation of Risk Evaluation Apparatus)

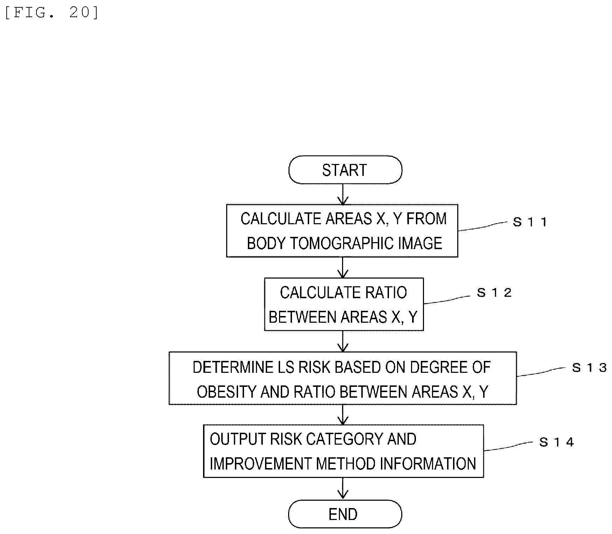

Hereinafter, operation of the risk evaluation apparatus 30 according to the third embodiment of the present invention will be described. FIG. 20 is a flowchart for describing the operation of the risk evaluation apparatus 30 according to the third embodiment of the present invention. Using the risk evaluation apparatus 30, one example of the risk evaluation method according to the present embodiment can be implemented. The flowchart of FIG. 20 corresponds to a risk evaluation program which is a program to be executed by the CPU (computer) of the risk evaluation apparatus 30.

First, in Step S11, the acquirer 101 calculates the visceral fat area X and the iliopsoas muscle area Y from a body tomographic image. The body tomographic image is, for example, a CT image of the position of the navel of a subject provided directly from the subject or via the healthcare facility. Specifically, the acquirer 101 causes the image processor to extract the visceral fat and the iliopsoas muscle from the CT image, and causes the arithmetic processor to calculate the visceral fat area X and the iliopsoas muscle area Y.

Subsequently, in Step S12, the acquirer 101 calculates the ratio between the areas X, Y. Specifically, the acquirer 101 causes the arithmetic processor to calculate, for example, X/Y.

Subsequently, in Step S13, the determiner 102 determines the LS risk based on the degree of obesity and the ratio between the areas X, Y. Specifically, the determiner 102 determines the LS risk based on the ratio (for example, X/Y) between the areas X, Y. Specifically, the determiner 102 causes the first comparator to compare the degree of obesity with the first threshold Th1, causes the second comparator to compare X/Y with the second threshold Th2, reads the risk category corresponding to the category indicator determined based on the comparison results from the first storage table 103a, and transmits the risk category to the output 104.

Finally, in Step S14, the output 104 reads the improvement method information adapted to the risk category from the determiner 102 from the second storage table 103b, and outputs such risk category and improvement method information.

According to the risk evaluation apparatus 30 of the third embodiment described above, effects similar to those of the risk evaluation apparatus 20 according to the second embodiment can be obtained, and the improvement method information is also provided to the subject, which can cause the subject to take specific measures against the LS risk.

Further, the risk evaluation program for operating the risk evaluation apparatus 30 and the risk evaluation method implemented using the risk evaluation apparatus 30 can also be provided.

### <4. Risk Evaluation Apparatus according to Fourth Embodiment of Present Invention>

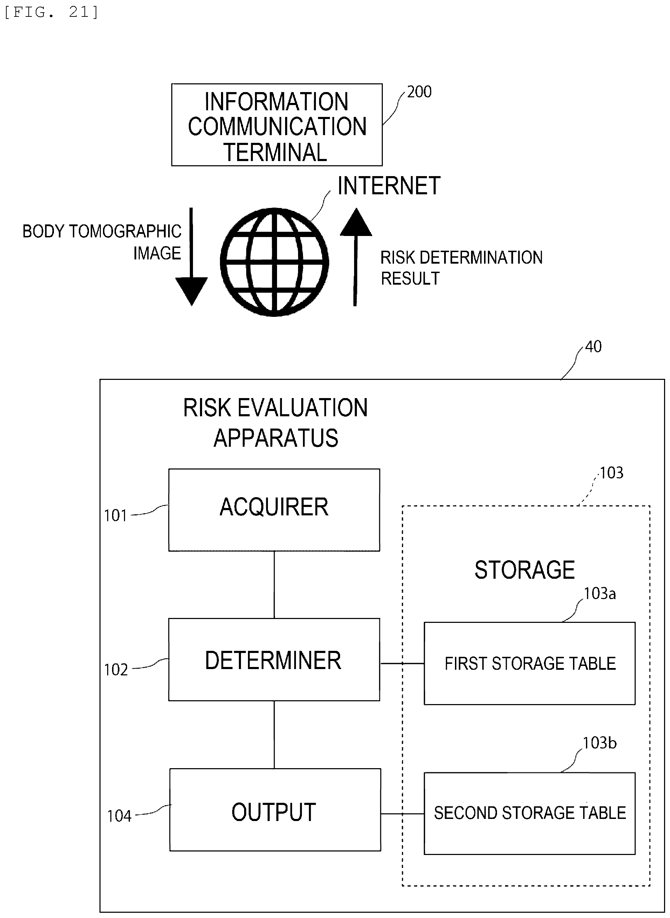

Hereinafter, a risk evaluation apparatus 40 according to a fourth embodiment of the present invention will be described with reference to FIG. 21. FIG. 21 is a block diagram showing the configuration of the risk evaluation apparatus 40 according to the fourth embodiment of the present invention.

The risk evaluation apparatus 40 has a configuration similar to that of the risk evaluation apparatus 30 according to the third embodiment, except that the risk evaluation apparatus 40 can perform transmission to and reception from an information communication terminal 200 (for example, personal computer, smartphone, or the like) via a network (for example, the Internet, the same also applies hereinafter). The risk evaluation apparatus 40 has an input-output interface connected to the network.

The risk evaluation apparatus 40 receives, via the network, the first information (for example, area X) and the second information (for example, area Y) or information (for example, body tomographic image) including the first information and the second information from, for example, the information communication terminal 200 of the subject or the healthcare facility as the representative of the subject. Examples of the information communication terminal 200 include a personal computer, a smartphone, and the like.

In the risk evaluation apparatus 40, the output 104 transmits the LS risk determination result obtained by the determiner 102 to, for example, the information communication terminal 200 of the subject or the healthcare facility via the network (for example, the Internet).

According to the risk evaluation apparatus 40 described above, the transmission and reception between the risk evaluation apparatus 40 and the information communication terminal 200 can be performed via the network, and therefore, the subject can make a request for the LS risk evaluation and receive the evaluation result without visiting to the healthcare facility or the like at which the risk evaluation apparatus 40 is placed. Note that only one of the transmission or the reception may be available between the risk evaluation apparatus 40 and the information communication terminal 200 via the network. For example, in a case where only the transmission is available, only the request for the LS risk evaluation can be made via the network, and for example, in a case where only the reception is available, only the reception of the LS risk evaluation result is available via the network.

Further, a risk evaluation program for operating the risk evaluation apparatus 40 and a risk evaluation method implemented using the risk evaluation apparatus 40 can also be provided.

### <5. Risk Evaluation Apparatus according to Fifth Embodiment of Present Invention>

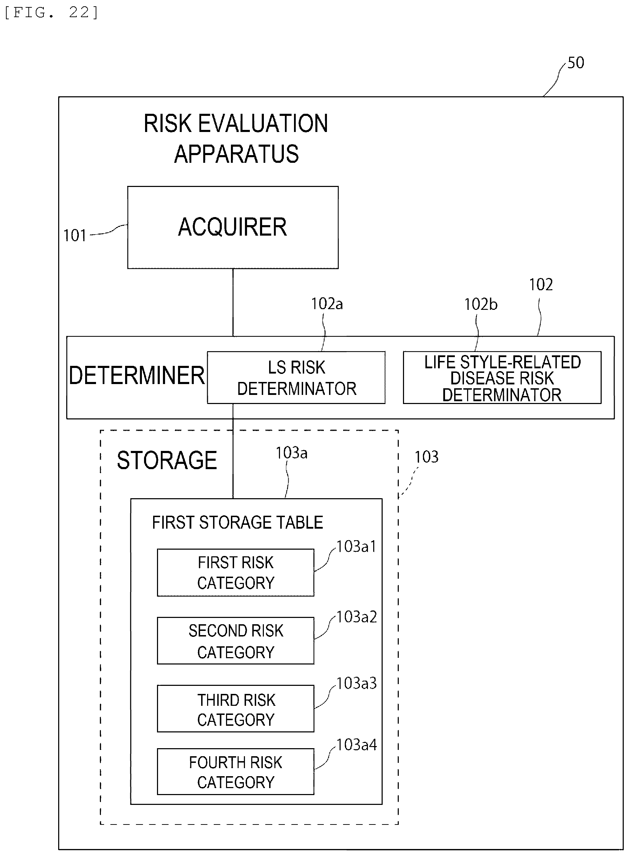

Hereinafter, a risk evaluation apparatus 50 according to a fifth embodiment of the present invention will be described with reference to FIG. 22. FIG. 22 is a block diagram showing the configuration of the risk evaluation apparatus 50 according to the fifth embodiment of the present invention.

The risk evaluation apparatus 50 has a configuration similar to that of the risk evaluation apparatus 20 according to the second embodiment, except that the determiner 102 not only determines the risk of the locomotive syndrome, but also determines the risk of a life style-related disease. Here, the life style-related disease includes diabetes, hyperlipidemia, a high-blood pressure, cancers, brain stroke, cardiac diseases, and the like.

In the risk evaluation apparatus 50, the determiner 102 has an LS risk determinator 102a and a life style-related disease risk determinator 102b.

Similarly to the determiner 102 of the risk evaluation apparatus 20 according to the second embodiment, the LS risk determinator 102a determines the LS risk based on the degree of obesity and X/Y, and outputs any of the first to fourth risk categories 103a1 to 103a4 as the LS risk determination result.

The life style-related disease risk determinator 102b outputs a determination result indicating a low life style-related disease risk, for example, in a case where the LS risk determinator 102a outputs the first risk category 103a1 as the LS risk determination result.

The life style-related disease risk determinator 102b outputs a determination result indicating an intermediate life style-related disease risk, for example, in a case where the LS risk determinator 102a outputs the second risk category 103a2 as the LS risk determination result.

The life style-related disease risk determinator 102b outputs a determination result indicating an intermediate life style-related disease risk, for example, in a case where the LS risk determinator 102a outputs the third risk category 103a3 as the LS risk determination result.

The life style-related disease risk determinator 102b outputs a determination result indicating a high life style-related disease risk, for example, in a case where the LS risk determinator 102a outputs the fourth risk category 103a4 as the LS risk determination result.

As described above, in the risk evaluation apparatus 50, the determiner 102 outputs the risk category as the LS risk determination result and the life style-related disease risk determination result corresponding to such a risk category.

Further, a risk evaluation program for operating the risk evaluation apparatus 50 and a risk evaluation method implemented using the risk evaluation apparatus 50 can also be provided.

### <6. Modifications of Present Invention>

The present invention is not limited to each embodiment above, and may be changed as necessary. For example, some of the configurations of the risk evaluation apparatuses according to the embodiments above may be combined without conflict therebetween.

In each embodiment above, the first information (for example, area X) correlated with the visceral fat mass and the second information (for example, area Y) correlated with the iliopsoas muscle mass are acquired from the body image information (for example, body tomographic image). Instead, for example, a belt-shaped energization/resistance measurement member may be wound around the position of the navel of the body, and power is supplied to the body to measure electrical resistance value distribution. In this manner, the visceral fat mass and the iliopsoas muscle mass may be acquired.

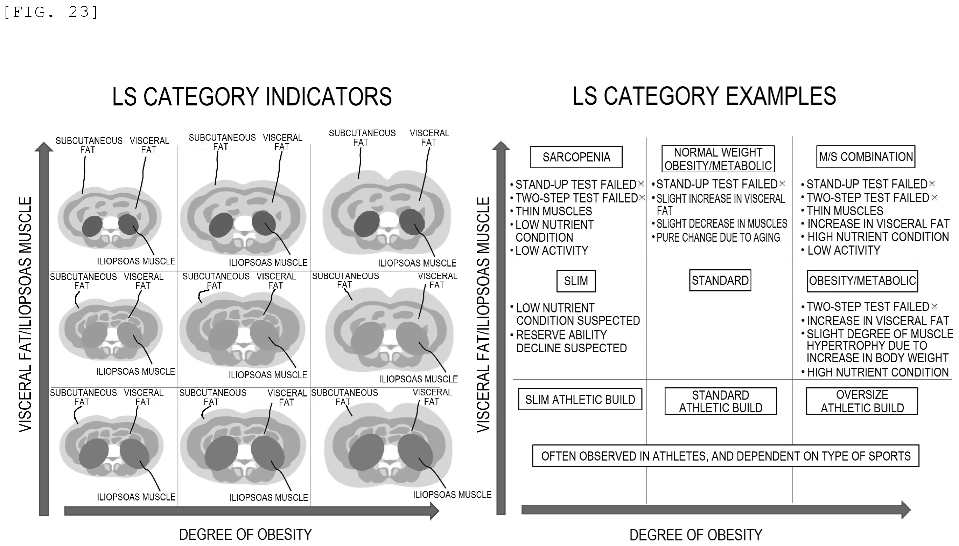

In the second, third, and fifth embodiments above, the four category indicators and the four LS categories are applied, but the present invention is not limited thereto and nine category indicators and nine LS categories may be applied, for example, as shown in FIG. 23. In FIG. 23, a graph on the left side shows the LS category indicators, and a graph on the right side shows LS category examples. In the example of FIG. 23, as shown in the graph on the left side in FIG. 23, there are five category indicators of (5) intermediate degree of obesity, low X/Y, (6) intermediate degree of obesity, intermediate X/Y, (7) intermediate degree of obesity, high X/Y, (8) low degree of obesity, intermediate X/Y, and (9) high degree of obesity, intermediate X/Y in addition to the four category indicators of (1) low degree of obesity, low X/Y, (2) high degree of obesity, low X/Y, (3) low degree of obesity, high X/Y, and (4) high degree of obesity, high X/Y. (1) low degree of obesity, low X/Y is adapted to a slim athletic build type of the graph on the right side in FIG. 23. (2) high degree of obesity, low X/Y is adapted to an oversize athletic build type of the graph on the right side in FIG. 23. (3) low degree of obesity, high X/Y is adapted to the sarcopenia type of the graph on the right side in FIG. 23. (4) high degree of obesity, high X/Y is adapted to the M/S combination type of the graph on the right side in FIG. 23. (5) intermediate degree of obesity, low X/Y is adapted to a standard athletic build type of the graph on the right side in FIG. 23. (6) intermediate degree of obesity, intermediate X/Y is adapted to a standard type of the graph on the right side in FIG. 23. (7) intermediate degree of obesity, high X/Y is adapted to a normal weight obesity/metabolic type of the graph on the right side in FIG. 23. (8) low degree of obesity, intermediate X/Y is adapted to a slim type of the graph on the right side in FIG. 23. (9) high degree of obesity, intermediate X/Y is adapted to an obesity/metabolic type of the graph on the right side in FIG. 23.

The LS risk can be determined and evaluated based on the visceral fat mass and the iliopsoas muscle mass of the body as described in each embodiment above, and therefore, it is assumed that this technical idea can be expanded and the LS risk can be determined and evaluated based on the fat mass and the muscle mass of the body.

For example, the LS risk may be determined and evaluated by measuring a balance between the fat mass and the muscle mass with a body composition monitor.

Thus, the LS risk was evaluated as follows with the body composition monitor.

### (Evaluation Target)

Persons (for example, 641 men and women) who got a comprehensive medical examination within one year and were subjected to the LS risk test and the measurement with the body composition monitor were targeted for evaluation of the LS risk.

### (Body Composition Monitor)

A multi-frequency body composition monitor (MC-780AN, TANITA Corporation) was used as the body composition monitor.

### (Measurement Items)

A body weight, a body composition, a body water percentage, a body water content, a lean body mass, an estimated bone mass, a body fat percentage, a visceral fat level, a muscle mass, an appendicular skeletal muscle mass, a right arm muscle mass, a left arm muscle mass, a right leg muscle mass, a left leg muscle mass, a total muscle mass score, a left-right arm balance, a left-right leg balance, a leg score, SMI [Appendicular Skeletal Muscle Mass (kg)/Body Height² (m²)], MM/H^2 [Muscle Mass (kg)/Body Height² (m²)], ASM/BW [Appendicular Skeletal Muscle Mass (kg)/Body Weight [kg]], MM/BW [Muscle Mass (kg)/Body Weight (kg)], a basal metabolic rate, and a basal metabolic rate assessment were measurement items.

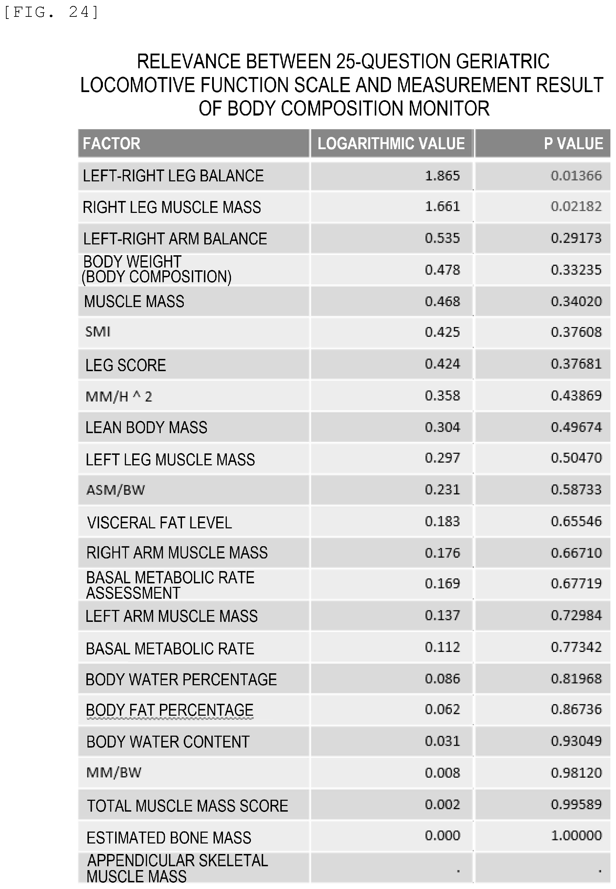

FIG. 24 is a table showing relevance between the 25-question geriatric locomotive function scale and the measurement results of the body composition monitor. FIG. 24 shows a logarithmic value and a P value for each factor (factor which may be a cause of the LS) obtained from the results of the measurement obtained on the body with the body composition monitor. In FIG. 24, as the logarithmic value for the factor increases and the P value for the factor decreases, the factor is more relevant to the 25-question geriatric locomotive function scale. FIG. 24 shows that among the factors obtained from the measurement results of the body composition monitor, the left-right leg balance and the right leg muscle mass are greatly relevant to the 25-question geriatric locomotive function scale. This suggests that if a left-right leg imbalance (specifically, left-right leg muscle mass imbalance) is corrected and/or the right leg muscle mass increases, the test result of the 25-question geriatric locomotive function scale may be improved.

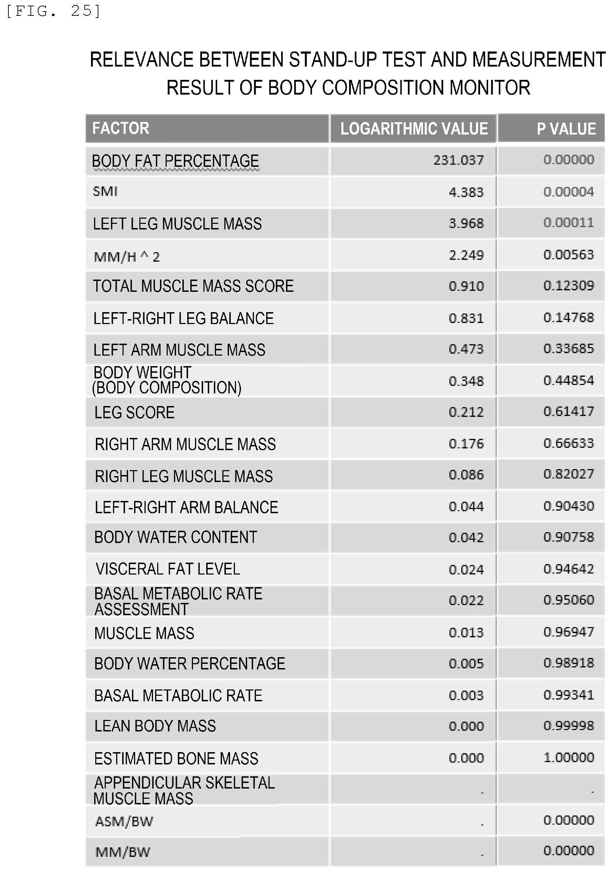

FIG. 25 is a table showing relevance between the stand-up test and the measurement results of the body composition monitor. FIG. 25 shows a logarithmic value and a P value for each factor (factor which may be a cause of the LS) obtained from the results of the measurement obtained on the body with the body composition monitor. In FIG. 25, as the logarithmic value for the factor increases and the P value for the factor decreases, the factor is more relevant to the stand-up test. FIG. 25 shows that among the factors obtained from the measurement results of the body composition monitor, the body fat percentage, the SMI, and the left leg muscle mass are greatly relevant to the stand-up test. This suggests that if the body fat percentage decreases, the SMI increases, and/or the left leg muscle mass increases, the test result of the stand-up test may be improved.

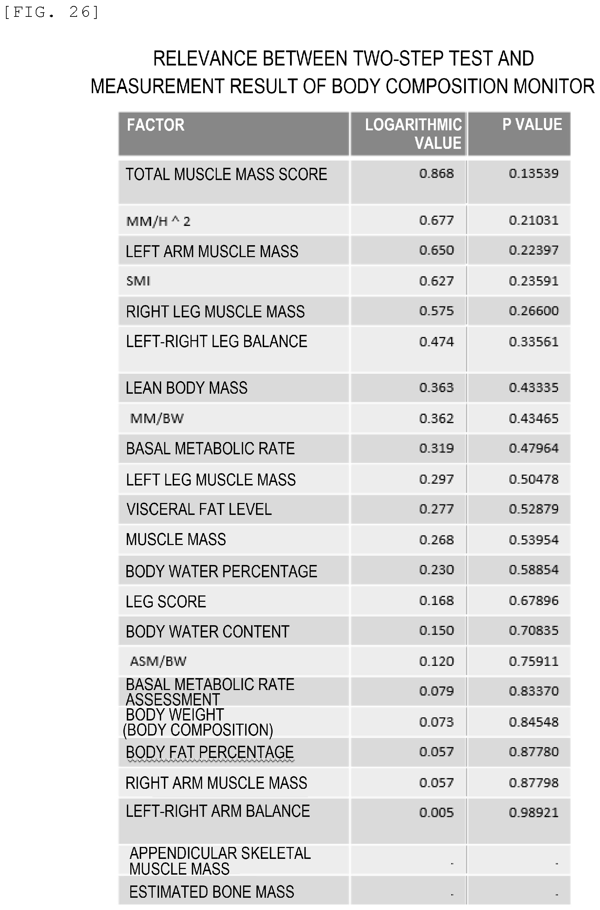

FIG. 26 is a table showing relevance between the two-step test and the measurement results of the body composition monitor. FIG. 26 shows a logarithmic value and a P value for each factor (factor which may be a cause of the LS) obtained from the results of the measurement obtained on the body with the body composition monitor. In FIG. 26, as the logarithmic value for the factor increases and the P value for the factor decreases, the factor is more relevant to the two-step test. FIG. 26 shows that any of the factors obtained from the measurement results of the body composition monitor is not greatly relevant to the two-step test.

From the analysis above, the measurement results of the body composition monitor include the factors (measurement items) greatly relevant to at least the 25-question geriatric locomotive function scale and the stand-up test of the LS risk test, and these factors can be used for determining and evaluating the LS risk. That is, the LS risk can be determined and evaluated based on information (first information) correlated with the fat mass of the body such as a body fat percentage and information (second information) correlated with the muscle mass of the body such as a lower limb muscle mass (right leg muscle mass and/or left leg muscle mass) and an SMI. Further, a risk evaluation apparatus, a risk evaluation program, and a risk evaluation method obtained by embodying this technical idea can be provided.

The risk evaluation apparatus is a risk evaluation apparatus including an acquirer that acquires first information correlated with the fat mass of a body and second information correlated with the muscle mass of the body, and a determiner that determines the risk (LS risk) of the locomotive syndrome based at least on the first information and the second information.

The acquirer may acquire the first information and the second information from a result of measurement obtained on the body with a body composition monitor. The determiner may determine the LS risk from a ratio between the first information and the second information. The first information may include a body fat percentage. The second information may include a leg muscle mass (muscle mass of one leg or muscle mass of both legs) and/or an SMI.

The risk evaluation program is a risk evaluation program causing a computer to execute a process of acquiring first information correlated with the fat mass of a body and second information correlated with the muscle mass of the body and a process of determining the risk (LS risk) of the locomotive syndrome based at least on the first information and the second information.

The acquiring process may acquire the first information and the second information from a result of measurement obtained on the body with a body composition monitor. The determining process may determine the LS risk from a ratio between the first information and the second information. The first information may include a body fat percentage. The second information may include a leg muscle mass (muscle mass of one leg or muscle mass of both legs) and/or an SMI.

The risk evaluation method is a risk evaluation method including a step of acquiring first information correlated with the fat mass of a body and second information correlated with the muscle mass of the body and a step of determining the risk (LS risk) of the locomotive syndrome based at least on the first information and the second information.

The acquiring step may acquire the first information and the second information from a result of measurement obtained on the body with a body composition monitor. The determining step may determine the LS risk from a ratio between the first information and the second information. The first information may include a body fat percentage. The second information may include a leg muscle mass (muscle mass of one leg or muscle mass of both legs) and/or an SMI.

### Reference Signs List

10, 20, 30, 40, 50: Risk Evaluation Apparatus
101: Acquirer
102: Determiner
103: Storage
103a1: First Risk Category (Category)
103a2: Second Risk Category (Category)
103a3: Third Risk Category (Category)
103a4: Fourth Risk Category (Category)
103b1: First Improvement Method Information (Improvement Method Information)
103b2: Second Improvement Method Information (Improvement Method Information)
103b3: Third Improvement Method Information (Improvement Method Information)
103b4: Fourth Improvement Method Information (Improvement Method Information)
104: Output

## Claims

1. A risk evaluation apparatus comprising:
an acquirer that acquires first information correlated with a fat mass of a body and second information correlated with a muscle mass; and
a determiner that determines a risk of a locomotive syndrome based at least on the first information and the second information.

2. The risk evaluation apparatus according to claim 1, wherein
a fat is a visceral fat, and
a muscle is an iliopsoas muscle.

3. The risk evaluation apparatus according to claim 2, wherein
the acquirer acquires the first information and the second information from body image information indicating an image of an internal structure of the body.

4. The risk evaluation apparatus according to claim 3, wherein
the body image information is a two-dimensional image obtained by imaging a predetermined position of the body,
the acquirer
calculates an area X of the visceral fat as the first information from the two-dimensional image, and
calculates an area Y of the iliopsoas muscle as the second information from the two-dimensional image, and
the determiner makes the determination based on the area X and the area Y.

5. The risk evaluation apparatus according to claim 4, wherein
the determiner makes the determination using an area ratio between the area X and the area Y.

6. The risk evaluation apparatus according to claim 4, wherein
the predetermined position is a position of a navel of the body.

7. The risk evaluation apparatus according to claim 4, wherein
the two-dimensional image is a body tomographic image obtained by computed tomography (CT).

8. The risk evaluation apparatus according to claim 5, wherein
the determiner categorizes the risk into a plurality of categories corresponding to a plurality of category indicators by the area ratio and a degree of obesity of the body, and determines the risk.

9. The risk evaluation apparatus according to claim 8, wherein
the plurality of category indicators include the following four category indicators:
(1) low degree of obesity, low X/Y;
(2) high degree of obesity, low X/Y;
(3) low degree of obesity, high X/Y; and
(4) high degree of obesity, high X/Y.

10. The risk evaluation apparatus according to claim 8, further comprising:
a storage that stores improvement method information on the risk, which is adapted to each of the plurality of categories,
wherein the improvement method information adapted to the category determined by the determiner is output.

11. The risk evaluation apparatus according to claim 1, wherein
the acquirer acquires the first information and the second information from a result of measurement obtained on the body with a body composition monitor.

12. The risk evaluation apparatus according to claim 11, wherein
the first information includes a body fat percentage.

13. The risk evaluation apparatus according to claim 11, wherein
the second information includes a leg muscle mass and/or an SMI.

14. The risk evaluation apparatus according to any one of claims 1 to 13, wherein
the first information and the second information or information including the first information and the second information are received via a network.

15. The risk evaluation apparatus according to any one of claims 1 to 13, wherein
a risk determination result is transmitted via a network.

16. The risk evaluation apparatus according to any one of claims 1 to 13, wherein
the determiner not only determines the risk of the locomotive syndrome, but also determines a life style-related disease risk.

17. A risk evaluation program causing a computer to execute
a process of acquiring first information correlated with a fat mass of a body and second information correlated with a muscle mass, and
a process of determining a risk of a locomotive syndrome based at least on the first information and the second information.

18. A risk evaluation method comprising:
a step of acquiring first information correlated with a fat mass of a body and second information correlated with a muscle mass; and
a step of determining a risk of a locomotive syndrome based at least on the first information and the second information.
